# EUROPEAN PATENT SPECIFICATION

(11) **EP 2 073 846 B1**
(45) Date of publication and mention of the grant of the patent: **05.10.2016**
(21) Application number: 07827225.9
(22) Date of filing: 18.10.2007
(51) Int. Cl.: A61K 45/06, A61K 31/4515, A61K 31/65, A61P 25/18

(54) **COMBINATIONS OF ANTIPSYCHOTIC DRUGS AND TETRACYCLINES IN THE TREATMENT OF PSYCHIATRIC DISORDERS**
KOMBINATIONEN VON ANTIPSYCHOTIKA UND TETRACYCLINEN ZUR BEHANDLUNG VON PSYCHIATRISCHEN ERKRANKUNGEN
COMBINAISONS DE MÉDICAMENTS ANTIPSYCHOTIQUES ET DE TÉTRACYCLINES DANS LE TRAITEMENT DES TROUBLES PSYCHIATRIQUES

(30) Priority: 19.10.2006 US 852646 P; 29.03.2007 WO PCT/IL2007/000414
(43) Date of publication of application: 01.07.2009
(73) Proprietor: MOR RESEARCH APPLICATIONS LTD., Petach Tikva 49170 (IL)
(72) Inventor: MENDLOVIC, Shlomo, 43579 Ra anana (IL); LEVKOVITZ, Yechiel, 55322 Kiryat Ono (IL)
(74) Representative: Straus, Alexander
(86) International application number: PCT/IL2007/001251
(87) International publication number: WO 2008/047365

(56) References cited:
- WO-A-01/24781
- WO-A2-03/005971
- LEE YOUNG-RAE; PARK KWANG-HYUN; LIN ZHAO-ZHEN; KHO YOUNG-JONG ET AL: "A calcium-calmodulin antagonist blocks experimental Vibrio vulnificus cytolysin-induced lethality in an experimental mouse model" INFECTION AND IMMUNITY, vol. 72, no. 10, October 2004 (2004-10), pages 6157-6159, XP002452330 ISSN: 0019-9567
- MIYAOKA ET AL: "Possible antipsychotic effects of minocycline in patients with schizophrenia" PROGRESS IN NEURO-PSYCHOPHARMACOLOGY & BIOLOGICAL PSYCHIATRY, OXFORD, GB, vol. 31, no. 1, 22 December 2006 (2006-12-22), pages 304-307, XP005813436 ISSN: 0278-5846
- LEVKOVITZ ET AL: "Minocycline, a second-generation tetracycline, as a neuroprotective agent in an animal model of schizophrenia" BRAIN RESEARCH, ELSEVIER, AMSTERDAM, NL, vol. 1154, 7 June 2007 (2007-06-07), pages 154-162, XP022108446 ISSN: 0006-8993
- BONELLI R M; NIEDERWIESER G; DIEZ J; KOELTRINGER P: "Riluzole and olanzapine in Huntington's disease" EUROPEAN JOURNAL OF NEUROLOGY, vol. 9, no. 2, March 2002 (2002-03), pages 183-184, XP002452331 ISSN: 1351-5101

## Description

### FIELD OF THE INVENTION

The present invention provides combined therapies for improved treatment of psychotic disorders. In particular, the present invention relates to combinations of an antipsychotic drug and a minocycline for use in the treatment of a first psychotic episode of schizophrenia.

### BACKGROUND OF THE INVENTION

### Schizophrenia

Schizophrenia is a disorder characterized by disturbances in perception, thought, volition, socialization, psychomotor behavior and the sense of self. Among clinicians and investigators there is little doubt that, in the majority of patients, schizophrenia runs a progressive course.

Patients suffering from schizophrenia start from a point of relative normalcy or subtle impairment. Following the formal onset most patients experience, to some degree, what has been called clinical deterioration. This deterioration is manifest by the development of and increasing severity and persistence of positive symptoms such as delusional behavior and/or psychotic episodes, negative symptoms such as diminished social and functional capacity, and cognitive impairment. The underlying causes and precise temporal boundaries of this clinical deterioration are not known; however, it is generally attributed to progression of the illness and associated with periods of active positive symptoms. The deterioration occurs primarily in the early stages of the illness and it is generally confined to the first 5 years after onset. It also does not progress in a linear or inexorable course to death or severe physical disability, as do classical neurodegenerative diseases. Rather, after a period, patients stabilize at a level where they have persistent symptoms and are impaired in their social and vocational function. After that point, additional exacerbations may occur but they are not usually associated with further deterioration.

Pharmacological animal models of schizophrenia in rodents utilize drugs that mimic symptoms of human schizophrenia and are an accepted standard for pre-clinical schizophrenia research. Dizocilpine maleate (MK801) is a non-competitive NMDA receptor antagonist used in such animal models. The administration of NMDA receptor antagonists to normal individuals produces a cluster of symptoms that include positive symptoms, negative symptoms and cognitive impairments.

### Minocycline and other tetracyclines

Minocycline is a second-generation tetracycline commonly used in humans, because of its beneficial anti-microbial and anti-inflammatory properties. It has an excellent brain tissue penetration, is clinically well tolerated and completely absorbed when taken orally (Aronson, J Am Vet Med Assoc. 1980; 176(10 Spec No):1061-8; Barza et al., Antimicrob Agents Chemother. 1975; 8(6):713-20.). In addition, it has demonstrated neuroprotective qualities in animal models of cerebral ischemia, traumatic brain injury (Wang et al., Brain Res. 2003; 963(1-2):327-9; Yrjanheikki et al., Proc Natl Acad Sci USA. 1999; 96(23):13496-500), Huntington's disease (Chen et al, Nat Med. 2000; 6(7):797-801) and Parkinson's disease (Wu et al., J Neurosci. 2002; 22(5):1763-71). The neuroprotective effect of minocycline may be associated with its inhibition of inducible NO synthase (iNOS), caspase 1 and caspase 3 expression, and p38 mitogen activated protein kinase (MAPK) (Amin et al., Proc Natl Acad Sci USA. 1996, 93(24):14014-9; Tikka et al., J Neurosci. 2001; 21(8):2580-8). Recently, it was found that minocycline also inhibits cytochrome c release and delays progression of amyotrophic lateral sclerosis in mice (Zhu et al., Nature. 2002; 417(6884):74-8). Furthermore, an additive neuroprotective effect was obtained when a combination of minocycline and creatine (another agent available for ALS treatment) was administered to mutant mice in an ALS model (Zhang et al., Ann. Neurol., 2003; 53:267-270).

Several patents disclose the use of tetracycline and tetracycline-derived compounds to treat disorders and injuries affecting the brain or the peripheral nervous system. US Pat. No. 6,613,756 disclosed the use of tetracycline derivatives to treat multiple sclerosis, wherein the symptoms of the disease were diminished in an animal model of the disease. US Pat. App. No. 2003/0092683 discloses the use of tetracyclines and tetracycline derivatives for treatment of Parkinson's disease and related disorders, and further discloses the use of tetracyclines and tetracycline derivatives as neuroprotective agents. US Pat. No. 6,649,589 discloses the use of tetracycline and tetracycline derivatives for treating nerve root injuries. US Pat. No. 6,319,910 teaches the use of chemically modified tetracyclines, which have no or negligible antimicrobial activity, for treating diseases or disorders associated with elevated levels of cyclooxygenase-2 (COX-2) and TNF-alpha, such as multiple sclerosis, septic shock, graft-vs.-host disease, cerebral malaria, cachexia associated with HIV infection, brain ischemia, inflammatory bowel disease, and neurodegenerative disorders. US Pat. No. 6,277,393 teaches a method for treating or suppressing brain or nerve injuries by administering tetracycline or tetracycline derivatives. All the abovementioned disclosures are based on the results of experiments performed on isolated cells or on animal models of the diseases, not on the results of human trials.

### Treatment options

There are currently two main types of antipsychotics in use, the typical antipsychotics and the atypical antipsychotics. All antipsychotic drugs tend to block D2 dopamine receptors in the brain, so the normal effect of dopamine release in the relevant synapses is reduced. Atypical antipsychotic drugs also block or partially block serotonin receptors (particularly 5HT2A,C and 5HT1A receptors). Antipsychotic drugs tend to improve the positive symptoms affecting schizophrenic patients, while they do not improve the negative symptoms, and only slightly improve the cognitive deficits. The biggest problem with the current treatment with atypical antipsychotics is the so-called "metabolic syndrome", which includes obesity, diabetes, and increased cholesterol levels (Newcomer JW., Clin. Ther. 2004; 26(12):1936-46; Shirzadi A, and Ghaemi, S., Harv. Rev. Psychiatry 2006; 14(3):152-64; Antai-Otong D., Perspect. Psychiatr. Care 2004; 40(2):70-2).

Clearly, the pharmacological treatment of schizophrenia needs to be improved to achieve amelioration of the patient's symptoms.

Anecdotal case reports of two schizophrenic patients treated with antipsychotics, that had entered a state of catatonic stupor, have been published recently (Miyaoka et al., Prog. Neuropsychopharmacol. Biol. Psychiatry 2007; 31(1):304-7). In both cases, minocycline was successfully used to treat the catatonic symptoms. Minocycline is not known to be of utility in the treatment of schizophrenia. Among other treatments that have been postulated to be potential future options in the treatment of schizophrenia, the anti-apoptotic effect of minocycline was considered (Lieberman et al., CNS Spectrums 2006; 11(4) suppl.1-13).

Lee Young-Rae et al. (Infection and Immunity 2004; 72(10):6157-9) describe cytolysin as factor in the pathogenesis of *Vibrio vulnificus.* It was concluded that a calcium-calmodulin antagonist blocks *V. vulnificus* cytolysin-induced lethality in a mouse model.

Nowhere in the background art has it been shown that a combination of antipsychotic drugs and tetracyclines may prove to be more effective in treating the symptoms of schizophrenic patients than the current accepted pharmacological treatments, consisting of antipsychotic drugs only.

There is an unmet medical need for improved therapies for treating schizophrenia.

### SUMMARY OF THE INVENTION

The present invention provides a novel combination of minocycline and a pharmaceutically effective amount of at least one antipsychotic drug for use in the treatment of a first psychotic episode of psychotic disorders. It is now disclosed that said agents act synergistically to achieve a positive outcome in psychotic subjects. Not only are positive symptoms reduced, negative symptoms hitherto considered untreatable are reduced by the combination therapy as well. Surprisingly, the cognitive outcome was improved significantly by the combination treatment. Thus, the present invention provides unique advantages compared to known therapies with antipsychotic drugs alone.

Moreover, it is now disclosed for the first time that patients treated with a combination therapy are less prone to weight gain than patients treated with an antipsychotic agent alone. A significantly higher proportion of the patients receiving the combination therapy either lost weight or kept the same weight as compared to the patients treated with an antipsychotic agent alone.

The present invention is based in part on the unexpected discovery that minocycline is more effective than haloperidol, a typical antipsychotic, in the reversal of cognitive deficits in an animal model of schizophrenia. Another unexpected finding on which the present invention is founded, is that minocycline treatment alone improves these cognitive deficits. Thus minocycline monotherapy is an effective treatment for psychotic disorders and particularly symptoms associated with schizophrenia.

The present invention also discloses for the first time the utility of minocycline as an adjuvant drug providing an improved outcome in human subjects experiencing a first psychotic episode.

Thus, without wishing to be bound by any particular theory or mechanism of action, minocycline may be used in combination with current antipsychotic drugs for the treatment of psychotic disorders, particularly schizophrenia. The present invention discloses that a combination of minocycline and an antipsychotic drug is more effective in the treatment of symptoms of schizophrenia than either a typical or atypical antipsychotic alone. Preferably the effects obtained by a combined therapy using minocycline in addition to known antipsychotic medications are additive. More preferably the effects of a combined therapy comprising minocycline and one or more antipsychotic medication are synergistic.

Moreover, the teachings of the present invention are advantageous over previously known methods for treatment of schizophrenic patients, inasmuch as said methods of administering an antipsychotic drug alone cannot improve negative symptoms altogether, whereas the combination therapy according to the present invention improves negative symptoms.

It is to be understood explicitly that the scope of the present invention encompasses all types of antipsychotic drugs within the context of the present invention.

It is to be further understood that the scope of the present invention encompasses psychotic disorders amenable to treatment with antipsychotic agents. Within the scope of this invention, psychotic disorders include: schizophrenia, bipolar disorder, mood disorders, post-traumatic disorder, eating disorders, obsessive-compulsive disorder, substance-related disorders, somatoform disorders, and micro psychotic episodes in personality disorders. In an exemplary embodiment the psychotic disorder is schizophrenia.

According to one aspect the present invention provides a pharmaceutical composition comprising as active ingredients a first component which is any antipsychotic drug approved for use in humans, and a second component which is tetracycline pharmaceutically active amount of minocycline for use in the treatment of a first psychotic episode of a psychotic disorder. In some embodiments, the first component is a typical antipsychotic. In other embodiments, the first component is an atypical antipsychotic. In a preferred embodiment, the second component is minocycline.

Typical antipsychotic drugs include, but are not limited to: haloperidol, chlorpromazine, chlorprothixene, fluphenazine, loxapine, mesoridazine, perphenazine, pimozide, thioridazine, thiothixene, trifluoperazine, and trifluopromazine. Atypical antipsychotic drugs include, but are not limited to: olanzapine (disclosed in US Pat No. 5,229,382), risperidone (disclosed in US Pat No. 4,804,663), clozapine, quetiapine (disclosed in US Pat No. 4,879,288), paliperidone, ziprasidone (disclosed in US Pat No. 4,831,031), and aripiprazole (disclosed in US Pat No. 4,734,416).

In some embodiments of this aspect of the present invention, the first component is a typical antipsychotic drug. In other embodiments, the first component is an atypical antipsychotic drug.

The minocycline can be administered in dosage amounts of about 10 mg/day to about 500 mg/day, preferably of about 50 mg/day to about 200 mg/day.

In another embodiment of this aspect of the present invention, the pharmaceutical composition is administered as a pharmaceutical formulation comprising said pharmaceutical composition and pharmaceutically acceptable excipients, carriers, and diluents, if necessary. In some embodiments the pharmaceutical formulation is in the form of tablets, chewable tablets, capsules, syrups, suspensions, solutions, intranasal sprays, suppositories, transdermal patches, or other types of pharmaceutical formulations.

In further embodiments, the pharmaceutical composition may comprise a modified release formulation including but not limited to a delayed release, sustained release (also known as long acting, extended release, or slow release), controlled release, or pulsed release formulations.

Modified release pharmaceutical compositions allow administration of a single daily dose of an antipsychotic drug selected from an atypical and a typical antipsychotic agent, together with a tetracycline able to cross the blood brain barrier, to provide an advantageous delivery of drug for the treatment of psychiatric disorders. Such modified release is therefore considered to be particularly useful for the delivery of antipsychotic drugs in combination with the tetracycline, for the treatment of psychiatric disorders, especially schizophrenia. Among the advantages of modified release dosage forms, it is anticipated that a single daily dose will provide improved patient compliance and reduced side effects.

In some embodiments, it is envisaged that the release of only the antipsychotic drug is modified. In other embodiments it is also envisaged that the release of only the tetracycline is modified. The remaining active agent would of course be subject to non-modified release.

Alternatively, the release of both the antipsychotic drug and the tetracycline is suitably modified.

Suitably, the modified release is delayed, pulsed or sustained release.

In one aspect the modified release is a delayed release.

Delayed release is conveniently obtained by use of a gastric resistant formulation such as an enteric formulation, such as a tablet coated with a gastric resistant polymer, for example Eudragit L100-55. Other gastric resistant polymers include methacrylates, cellulose acetate phthalate, polyvinyl acetate phthalate, hydroxypropyl methylcellulose phthalate, in particular, Aquateric, Sureteric, HPMCP-HP-55S.

The enteric coated tablet may be a single layer tablet, where the active agents are admixed prior to compression into tablet form, or a multi-layer tablet, such as a bi-or tri-layer tablet, wherein each active agent is present in a discrete layer within the compressed tablet form. The discrete table layers can be arranged as required to provide modified or non-modified release of each active agent.

In a further aspect the modified release is a sustained release, for example providing effective release of active agents over a time period of up to 24 hours, typically in the range of 4 to 24 hours.

Sustained release is typically provided by use of a sustained release matrix, usually in tablet form, such as disintegrating, non-disintegrating or eroding matrices.

Sustained release is suitably obtained by use of a non disintegrating matrix tablet formulation, for example by incorporating Eudragit RS into the tablet. Alternative non disintegrating matrix tablet formulations are provided by incorporating methacrylates, cellulose acetates, hydroxypropyl methylcellulose phthalate, in particular Eudragit L and RL, Carbopol 971P, HPMCP-HP-55S into the tablet.

Sustained release is further obtained by use of a disintegrating matrix tablet formulation, for example by incorporating methacrylates, methylcellulose, in particular Eudragit L, Methocel K4M into the tablet.

Sustained release can also be achieved by using a semi-permeable membrane coated tablet for example by applying methacrylates, ethylcellulose, cellulose acetate, in particular Eudragit RS, Surelease to the tablet.

Sustained release can also be achieved by using a multi layer tablet, where each active ingredient is formulated together or as a separate layer, for example as a matrix tablet, with the other layers providing further control for sustained release of either one or both active agents.

Sustained release can also be achieved by using implants.

In yet a further aspect the modified release is a pulsed release, for example providing 2 up to 4, pulses of active agent per 24 hours.

One form of pulsed release is a combination of non-modified release of active agent and delayed release.

Suitable modified release includes controlled release. The composition of the invention also envisages a combination of pulsed, delayed and/or sustained release for each of the active agents, thereby enabling for example the release of the reagents at different times. For example, where the composition comprises an antipsychotic drug and a tetracycline, such as minocycline, the composition can be arranged to release the minocycline overnight.

A suitable typical antipsychotic is haloperidol. Other suitable typical antipsychotic drugs are chlorpromazine, chlorprothixene, fluphenazine, loxapine, mesoridazine, perphenazine, pimozide, thioridazine, thiothixene, trifluoperazine, and trifluopromazine.

Suitable atypical antipsychotic drugs include olanzapine, risperidone, clozapine, quetiapine, paliperidone, ziprasidone, and aripiprazole.

It will be understood that the antipsychotic drugs and the tetracycline are in a pharmaceutically acceptable form, including pharmaceutically acceptable derivatives such as pharmaceutically acceptable salts, esters and solvates thereof, as appropriate to the relevant pharmaceutically active agent chosen. In certain instances herein the names used for the antipsychotic drug or the tetracycline may relate to a particular pharmaceutical form of the relevant active agent: it will be understood that all pharmaceutically acceptable forms of the active agents per se are encompassed by this invention.

Suitable pharmaceutically acceptable forms of the antipsychotic drugs and the tetracycline depend upon the particular agent used but included are known pharmaceutically acceptable forms of the particular agent chosen.

The pharmaceutical composition can be administered in the form of a modified release formulation wherein the antipsychotic drug is released substantially before the tetracycline.

Orally administrable formulations such as tablets, chewable tablets, and capsules are preferred.

The pharmaceutical formulation can be administered to a subject orally, transdermally, percutaneously, intravenously, intramuscularly, intranasally, intrarectally or through other therapeutical routes of administration.

The administration may also occur at fixed intervals or at variable intervals. Alternatively, the administration may occur substantially concurrently or sequentially.

The daily dose of minocycline can be administered in a single dose once a day, or in smaller doses at least twice daily at fixed or variable intervals.

In preferred embodiments, the psychotic disorder is schizophrenia. The subject to be treated is for example a human being.

The first and second unit dosage forms may be contained in a single container. In an alternative, the first unit dosage form and the second unit dosage form are contained in separate containers.

The patient can be an antipsychotic drug-naïve patient.

Other objects, features and advantages of the present invention will become clear from the following description and drawings.

### BRIEF DESCRIPTION OF THE FIGURES

Figure 1 shows that minocycline pretreatment reverses the negative effect of MK801 on spatial learning and memory in a dose-dependent manner in the Morris Water Maze (MWM) task.
Figure 2 shows that minocycline pretreatment reverses the negative effect of MK801 on spatial learning and memory earlier than haloperidol treatment in the Morris Water Maze (MWM) task.
Figure 3 shows that cognitive deficits induced by MK801 in the Acoustic Startle Response task are prevented by minocycline pretreatment but not by haloperidol treatment.
Figure 4 shows that cognitive deficits induced by MK801 in the Pre Pulse Inhibition task are prevented by minocycline pretreatment but not by haloperidol treatment.
Figures 5-12 show the results of computerized tests and clinical assessments performed in a comparative study of human subjects experiencing a first psychotic episode and treated with an atypical antipsychotic and minocycline or placebo as an adjuvant treatment.

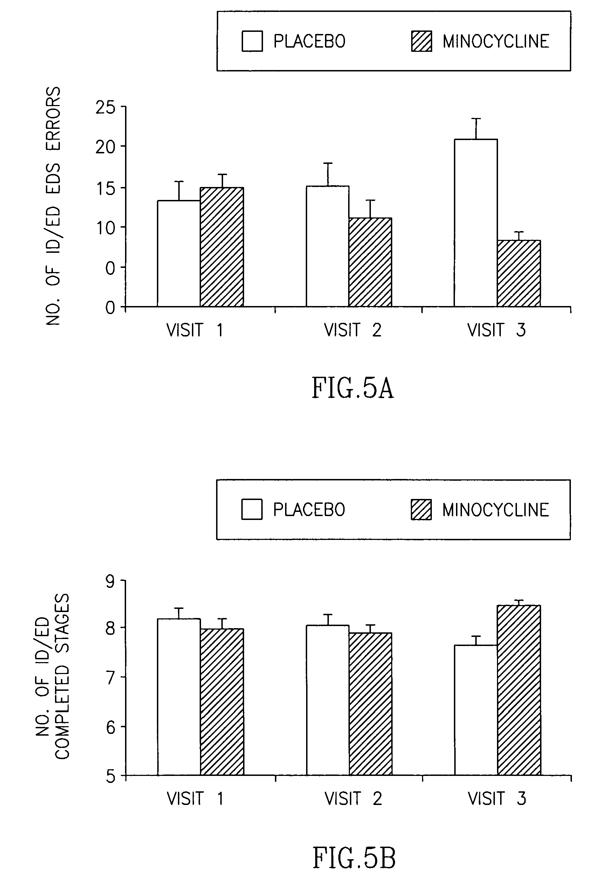

Figure 5 depicts the results of an Intra-extra dimensional set-shifts (IED) test. The task assesses rule acquisition and attentional set shifting abilities.

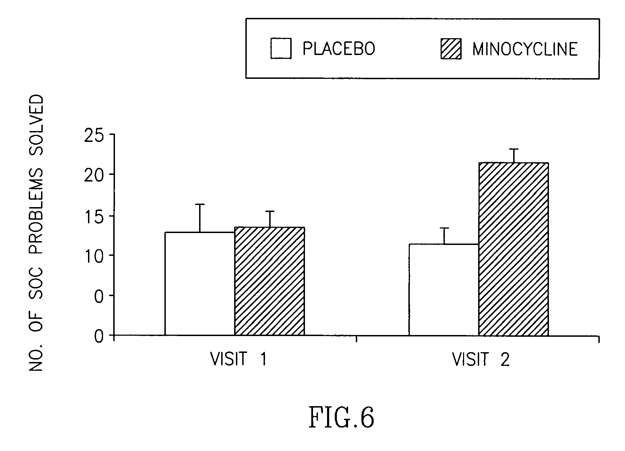

Figure 6 depicts the results of a Stockings of Cambridge (SOC) test. This spatial planning test gives a measure of frontal lobe function.

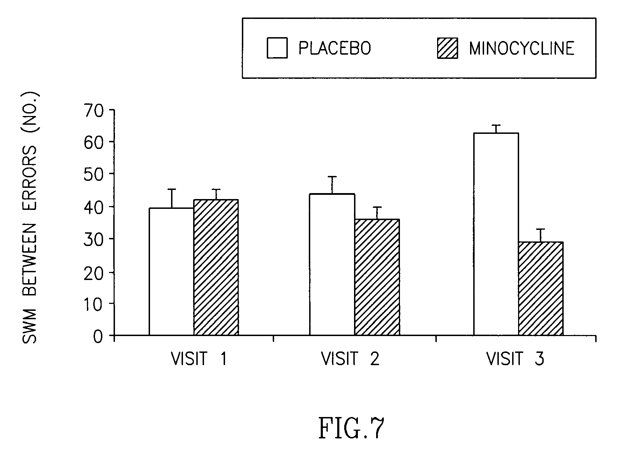

Figure 7 depicts the results of a Spatial Working Memory (SWM) test. The task has been linked to the frontal lobe and its sub-divisions (such as the dorsolateral and ventrolateral frontal), and may be considered an additional test of frontal activity.

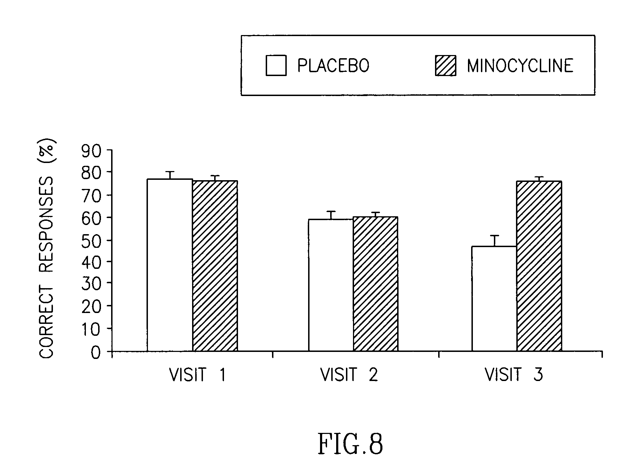

Figure 8 depicts the results of a Spatial Recognition Memory (SMR) test. The task tests spatial recognition memory.

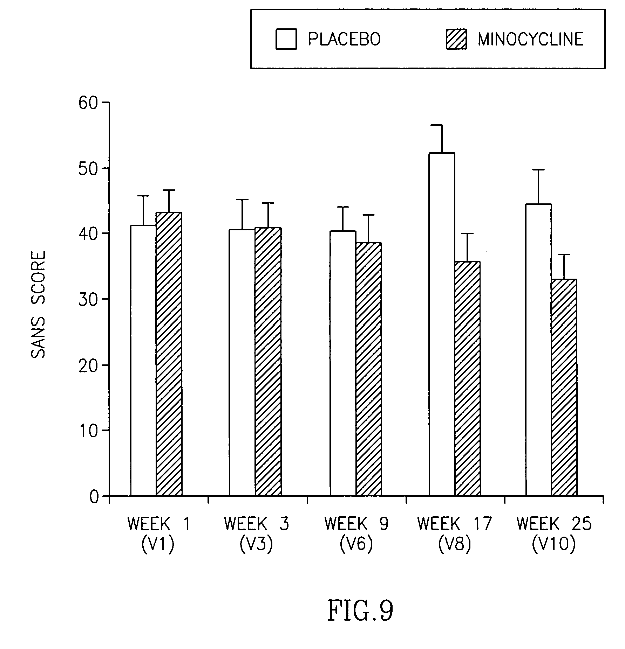

Figure 9 shows a Scale for Assessment of Negative Symptoms (SANS) evaluation of the negative symptoms of schizophrenia in the minocycline group compared to the placebo group. A higher score indicates a lower performance.

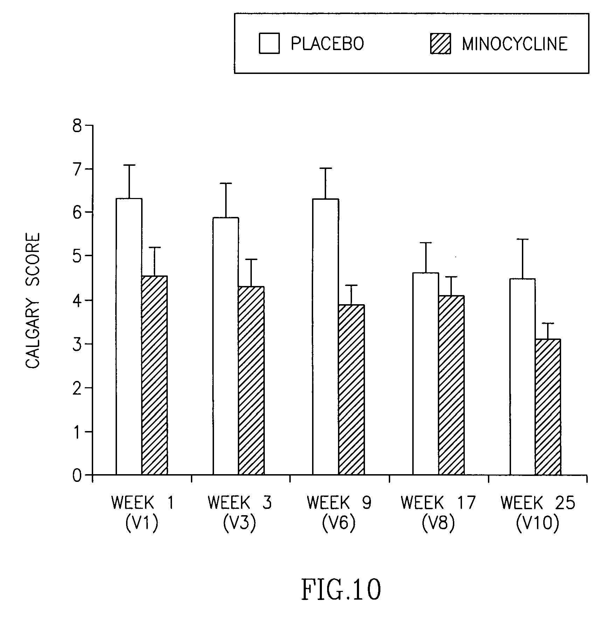

Figure 10 shows the Total Calgary Score in the minocycline group compared to the placebo group. This scale assesses the level of depression in schizophrenic patients. A higher score indicates a lower performance.

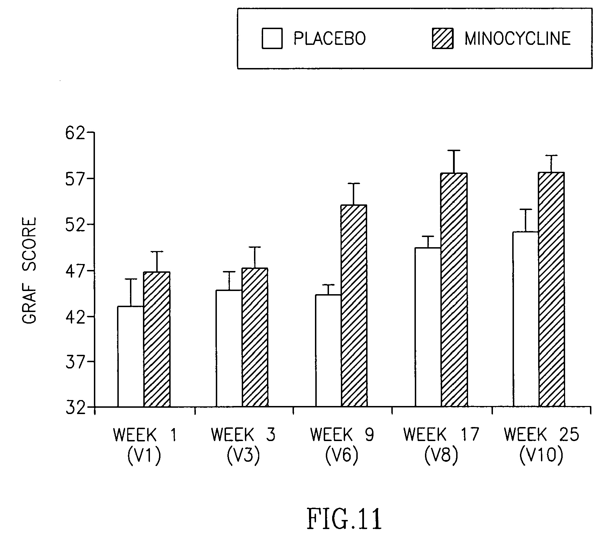

Figure 11 shows the GRAF score in the minocycline group compared to the placebo group. This scale is a measure of functional impairment in schizophrenic patients.

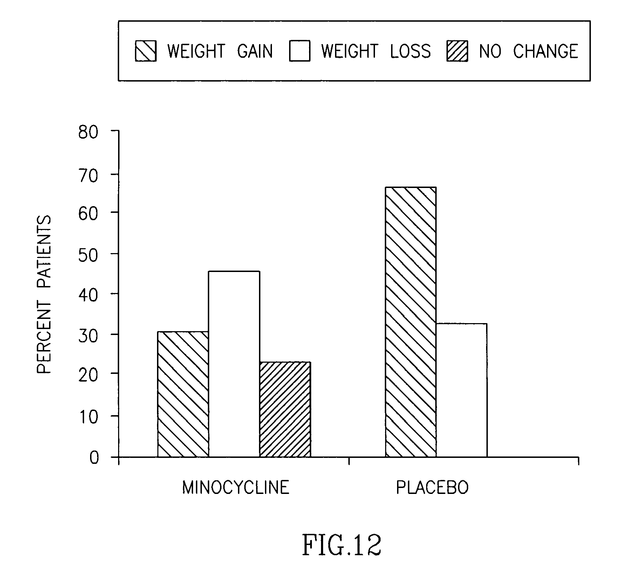

Figure 12 shows the distribution of body weight change in the minocycline group compared to the placebo group measured 12 weeks after start of treatment.

### DETAILED DESCRIPTION OF THE INVENTION

The present invention discloses the use of a combination of an antipsychotic drug and the tetracycline minocycline for the treatment of a first psychotic episode of psychotic disorders, particularly schizophrenia.

### Definitions

The term "schizophrenia" as used herein refers to a neuropsychiatric disorder in which the patient suffers from distorted thinking, hallucinations, and a reduced ability to feel normal emotions.

The term "positive symptoms" as used herein refers to the presence of distinctive behaviors in a schizophrenic patient, such as, but not limited to, strange or paranoid delusions, hallucinations, and fearful reaction to ordinary sights.

The term "negative symptoms" as used herein refers to the absence of normal social and interpersonal behaviors in a schizophrenic patient.

The term "cognitive deficits" as used herein refers to reduced or impaired psychological functions such as memory, attention, problem-solving, executive function or social cognition.

The term "prodromal" as used herein refers to a stage in the development of schizophrenia anterior to the syndromal level and defined by the appearance of nonspecific, early warning signs.

The terms "pharmaceutically effective amount" or "therapeutically effective amount" as used herein are used interchangeably and refer to the amount of active ingredient or active component in a pharmaceutical composition that will achieve the goal of treating or preventing symptoms of schizophrenia.

The term "combined therapy" as used herein refers to the combination of at least two therapeutic compounds for treating a patient with a disease in need of such treatment.

The term "controlled release" as used herein refers to a dosage form which, due to its special technological construction, provides for drug release having kinetics of zero order, at a sufficient rate to maintain the desired therapeutic level over an extended period of time. Also used to denote sustained release products in general (not necessarily limited to zero order).

The term "delayed release" as used herein refers to a dosage form that releases the drug(s) at a time other than promptly after administration. It is typically related to enteric coated tablets.

The term "sustained release" as used herein refers to a dosage form designed to release the drug(s) contained therein at a continuous and controlled rate for a longer period of time that can normally be achieved with its conventional, non-sustained counterpart. Consequently, peroral administration of a single dose of a sustained release product increases the duration of therapeutic action of the drug, beyond that achieved normally with a single dose of the corresponding non-sustained conventional counterpart. Other terms used to describe the same concept include: "controlled release", "extended-release" "long-acting", "gradual release", "prolonged action", and "slow release".

The term "pulsed release" as used herein refers to a dosage form designed to release the drug(s) contained therein in two or more separate pulses.

The term "implants" as used herein refers to small sterile polymeric matrices, pellets or particles for insertion or implanting into the body by surgical means or by injection to help achieving sustained release.

The term "antipsychotic drug-naive patient" as used herein means that the patient has never been treated with an antipsychotic drug, including, but not limited to, typical and atypical antipsychotic drugs.

The present invention is directed to the treatment of psychotic disorders. The term "psychotic disorder", as used herein, refers to any disorder amenable to treatment with antipsychotic drugs Examples of such disorders include, but are not limited to, schizophrenia, bipolar disorder, mood disorders, post-traumatic disorder, eating disorders, obsessive-compulsive disorder, substance-related disorders, somatoform disorders, and micro psychotic episodes in personality disorders. The present invention is especially directed to the treatment of schizophrenia.

The present invention provides a pharmaceutical composition for use in treating a first psychotic episode of a psychotic disorders. The composition comprises two active components, the first component being any antipsychotic drug approved for use in humans and the second component being the tetracycline minocycline. In a preferred embodiment, the disorder is schizophrenia.

In one embodiment, the first component is a typical antipsychotic drug. In another embodiment, the first component is an atypical antipsychotic drug.

The typical antipsychotic drugs include, but are not limited to: haloperidol, chlorpromazine, chlorprothixene, fluphenazine, loxapine, mesoridazine, perphenazine, pimozide, thioridazine, thiothixene, trifluoperazine, and tufluopromazine. Long acting, extended-release formulations of haloperidol, chlorprothixene, and fluphenazine are also used in the treatment of psychotic disorders. It is to be understood that this list is meant to give examples of representative typical antipsychotics and is not restrictive in the context of the present invention.

The atypical antipsychotic drugs include, but are not limited to: olanzapine, risperidone, clozapine, quetiapine, paliperidone, ziprasidone, and aripiprazole. Long acting, extended-release formulations of olanzapine and risperidone are also used in the treatment of psychotic disorders. It is to be understood that this list is meant to give examples of representative atypical antipsychotics and is not restrictive in the context of the present invention.

The chemical structure of tetracycline is a compound of formula I

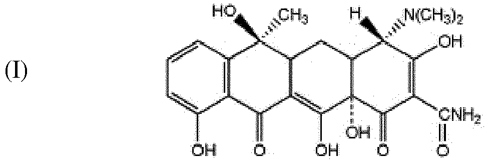

The tetracyclines are a family of drugs derived from the chemical structure of tetracycline, including but not limited to aureomycine, terramycine, demethylchlortetracycline, rolitetracycline, methacycline, doxycycline, glycylcyclines, and minocycline. The structure of minocycline is shown in Formula II.

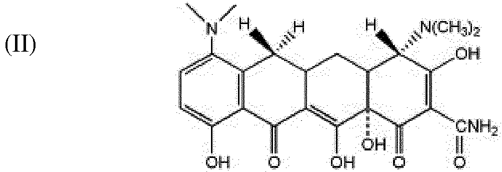

It will be understood that while the use of a single typical antipsychotic as a first component compound is preferred, combinations of two or more typical antipsychotics may be used as a first component if necessary or desired. Similarly, while the use of a single atypical antipsychotic as a first component compound is preferred, combinations of two or more atypical antipsychotics may be used as a first component if necessary or desired. Similarly, while the use of a single tetracycline as a second component compound is preferred, combinations of two or more tetracyclines may be used as a second component if necessary or desired.

The present invention, without wishing to be bound by theoretical considerations or by experimental results, points to the beneficial effect of minocycline on cognitive deficits and negative symptoms of schizophrenia. Therefore the combination comprises minocycline as the second component compound.

The choice of antipsychotic depends, in final analysis, on the clinical history and characteristics of the patients, and will be decided upon by the physician in charge of the case. Generally speaking, atypical antipsychotics are the first line agents for patients with new onset of a chronic psychotic disorder because they are associated with fewer extrapyramidal side effects, less propensity for the development of tardive diskynesia, and higher compliance than the typical antipsychotics. Therefore, a preferred embodiment of the present invention comprises at least one atypical antipsychotic as the first component compound. A currently preferred embodiment combination may comprise an atypical antipsychotic and minocycline.

An olanzapine/minocycline combination is an example of such a currently preferred embodiment in which the first component is an atypical antipsychotic and the second component is minocycline.

The dosage amounts used in the present invention vary with the type of medication and the clinical condition of the patient, and are to be determined by the professional caregiver. For the antipsychotic component of the formulation, the dosage will usually correspond to the dosage prescribed in accepted medical practice for treating a specific psychotic disorder with that specific drug.

For the tetracycline component of the formulation, the effective dosage remains to be determined. The results of the study disclosed in the present invention show that a daily dose of 200 mg of minocycline as an adjuvant drug is effective in treating certain negative symptoms of schizophrenia. This is also the daily dose of minocycline used to treat bacterial infections. The effectiveness of lower or higher minocycline dosages has not been ascertained and will be the object of further studies. In general, the dosage of the tetracycline component of the formulation will usually correspond to the pharmaceutically effective dosage used to treat bacterial infections that is considered to be safe for long-term use.

Accordingly, minocycline may be administered in dosage amounts of about 10 mg/day to about 500 mg/day, and preferably of about 50 mg/day to about 200 mg/day.

The daily dose of minocycline can administered in a single dose once a day or in several smaller doses at least twice daily at fixed or variable intervals.

For example, for an adult patient suffering from schizophrenia, an antipsychotic of choice is olanzapine, used at a dose of 15 mg/day. Such a patient may be treated with a formulation comprising 15 mg olanzapine and 200 mg minocycline, to be administered once a day. Other dosages and multiple daily administrations can be considered.

All the components in the aforementioned compositions can be administered orally. However, oral administration is not the only possible route. The formulation may be administered transdermally, percutaneously, intravenously, parenterally, intramuscularly, intranasally, or intrarectally, limited only by the physical properties of the components in the combination and by the convenience of the patient and the caregiver. For example transdermal administration may be considered for forgetful patients. Administration by injection of a solution may be desirable for patients who are adverse to treatment.

The formulation may take any physical form that is pharmaceutically acceptable. Dosage forms suitable for oral administration are particularly preferred. Such formulations contain an effective amount of each of the compounds, which effective amount is related to the daily dose of the compounds to be administered. The amounts of each drug to be contained in each dosage unit depend on the identity of the drugs present in the formulation. The inert ingredients and manner of formulation of the adjunctive pharmaceutical compositions are conventional, except for the presence of the combination of the present invention.

All of the known types of formulations may be used, including tablets, chewable tablets, capsules, solutions, parenteral solutions, intranasal sprays or powders, troches, suppositories, transdermal patches, suspensions, and implants.

For example, capsules are prepared by mixing the antipsychotic component and the tetracycline component of the combination with a suitable diluent and filling the proper amount of the mixture in the capsule. Alternatively, capsules are prepared in such way as the antipsychotic component is separated from the tetracycline component by a membrane or any other pharmaceutically acceptable physical means.

### Oral formulations for combination therapy

An oral formulation for combination therapy of minocycline and antipsychotic drug comprises a mixture of granules of minocycline and granules of antipsychotic drug, filled into a capsule, pressed into a tablet or suspended in a liquid, ready made or concentrated for reconstitution. The term "granule" used herein also includes particulate material such as spheres or pellets.

Minocycline and the antipsychotic drugs are separately wet-granulated and dried to obtain two types of granules: minocycline granules and antipsychotic drug granules. The granules are then compressed into tablets or filled into capsules in the desired proportions and doses. Granule compositions are easily amended by the skilled in the art to obtain desired size, flow properties and release profiles. Granules have the advantage of free flowing and accurate dose proportion control and suitability for modern mass production methods and pharmaceutical machinery.

The granules or pellets are prepared in a classical manner of wet or dry granulation described in the art (Remington's Pharmaceutical Sciences, 18th ed, 1990; Mack Publishing Company, Easton, Pa.). The granules are prepared in various compositions and are designed to be soft or hard, immediate- or delayed-release, according to the desired pharmacokinetic properties.

The separate granulation of minocycline and of the antipsychotic drug enables physical separation to avoid potential chemical incompatibilities. Encapsulation of granules, minocycline granules and/or antipsychotic granules, may serve as release-profile modulating method and a separation method to avoid chemical incompatibilities.

Granule preparation is described hereinbelow. The granules may comprise each drug separately or in combination in the same granule, in such a way as to form three types of granules: a) minocycline alone granules, b) antipsychotic drug alone granules, and c) minocycline/antipsychotic drug mixture in the same granule.

The minocycline, the antipsychotic drug, or the minocycline/antipsychotic drug mixture is blended in a planetary mixer with excipients, such as filler and granule binder and formed into a wet mass with water. The wet mass is granulated with an industrial extrusion granulator or spheronizing device and dried with hot air, preferably by a fluidized bed drier.

An oral formulation made of granules in a granulation process comprises: (1) forming a powder blend of the active ingredient with excipients, wherein said active ingredient is minocycline, antipsychotic drug, or minocycline/antipsychotic drug mixture, (2) wet granulating the powder blend with water to form granules, (3) drying the granules to remove the water, and (4) compressing the dried granules mixture into a desired tablet form or filling into capsules or bottles for reconstitution into a liquid syrup or sweetened suspension.

The wet granulation process helps form agglomerates of powders. These agglomerates are called "granules" or "pellets". A wet granulated formulation of minocycline and antipsychotic drug and process thereof are also envisioned. The granules of separate drugs enable specific processing and formulation tailored for the specific antipsychotic drug and for the minocycline, after combining the granules of different drugs into one unit dosage form. The separate granulation also makes it possible to design a different release profile for the different drugs in the combination therapy. For example, the separate granulation process enables one drug of the combination to have an immediate release profile and the other an extended release profile.

Wet-granulated formulations include an agent called a "binder," which, in contact with water, swells or starts dissolving, giving the mixture a gel-like consistency. In wet granulation, the drugs and excipients including the binder are formed into a dough mass with the aid of a solvent, preferably water or hydro alcoholic solvents. Traditionally, starch, starch paste, gelatin, and cellulosics such as hydroxypropylmethyl cellulose, hydroxyethyl cellulose, hydroxypropyl cellulose, microcrystalline cellulose, such as Avicel PH101, and polyvinyl pyrrolidone are used as binding agents in wet granulation formulations (Remington's Pharmaceutical Sciences, 18th ed, Mack Publishing Company: Easton, Pa., 1990, pp. 1635-36).

The Handbook of Drug Excipients, 2nd. Ed., edited by A. Wade and P. J. Weller. 1994, pages 223, 229 and 392, and many other pharmaceutical references describe the common use of water soluble polymers such as HPMC, HPC-L, and PVP as binders, either as wet binders or dry binders, in immediate and sustained release tablet formulations.

For non-sustained release applications, these binders are used in amounts that do not exceed 5%. Higher amounts are not recommended due to retardation of the dissolution rate for many drugs. Amounts higher than 5%, especially of HPMC, are commonly used for sustained release dosage forms, and are generally of high molecular weight grades.

The granules are further compressed into tablets with the aid of fillers, binders, lubricants and disintegrants. The disintegrant may be one of several modified starches or modified cellulose polymers. Croscarmellose sodium is particularly preferred. Croscarmellose sodium NF Type A is commercially available under the trade name "Ac-di-sol". Preferred lubricants include magnesium stearate, calcium stearate, stearic acid, surface active agents such as sodium lamyl sulfate, propylene glycol, sodium dodecane sulfonate, sodium oleate sulfonate, sodium laurate mixed with stearates and talc, and sodium stearyl fumarate. Magnesium stearate is especially preferred.

Preferred tablet or capsule diluents include: lactose, microcrystalline cellulose, calcium phosphate(s), mannitol, powdered cellulose, and pregelatinized starch. Lactose and microcrystalline cellulose are especially preferred. In particular, Avicel PH101, the trademarked name for microcrystalline cellulose NF manufactured by FMC Corp. is particularly preferred.

The tablet or capsule may be further entero-coated or designed for immediate or extended release as well as for dissolution in the mouth for waterless consumption. Preferred tablets are sugar coated and round oval caplets that are easy to swallow. Preferred capsules are rounded and easy to swallow. Preferred tablets or capsules are flash tablets or tablets that dissolve in the mouth or fast disintegrating tablets which are easier to administer and swallow even without water.

Also disclosed but not forming part of the present invention is a process for the preparation of a tablet containing minocycline and an antipsychotic drug as active ingredients for immediate release, which process comprises: (1) forming a powder blend of the active ingredients each one alone or in combination, with added diluents and binder, using a mixer such as a planetary or high shear granulator, (2) wet granulating the powder blend by addition of water while mixing and spheronizing and sieving or extrusion to form granules, (3) drying the granules with heated air for 10 minutes to 24 hours in a dryer (fluid bed or tray type), (4) milling the dried granules to a uniform size, (5) adding and blending a disintegrant to the dried milled particles, (6) adding and blending a lubricant to the mixture containing the disintegrant for 30 seconds to 20 minutes, and (7) compressing the lubricated granule mixture into a desired tablet form. The shape of the tablet is not critical.

Also disclosed but not forming part of the present invention is a process for the preparation of a tablet containing minocycline and an antipsychotic drug as active ingredients for extended release, which process comprises: (1) forming a powder blend of the active ingredient each one alone or in combination, with added diluents and binder, using a mixer such as a planetary or high shear granulator, (2) wet granulating the powder blend by the addition of water while mixing and spheronizing and sieving or extrusion to form granules, (3) drying the granules with heated air for 10 minutes to 24 hours in a dryer (fluid bed or tray type), (4) adding and blending a slow release matrix forming polymer to the dried granules, (5) adding and blending a lubricant to the mixture containing the dried milled particles, and (6) compressing the granule mixture into a desired tablet form. The shape of the tablet is not critical.

Also disclosed but not forming part of the present invention is a process for the preparation of a tablet containing minocycline and an antipsychotic drug as active ingredients for slow release, which process comprises: (1) forming a powder blend of the active ingredient each one alone or in combination, with added diluents and binder, using a mixer such as a planetary or high shear granulator, (2) wet granulating the powder blend by the addition of water while mixing and spheronizing and sieving or extrusion to form granules, (3) drying the granules with heated air for 10 minutes to 24 hours in a dryer (fluid bed or tray type), (4) adding and blending a lubricant to the mixture containing the dried milled particles, (5) compressing the granule mixture into a desired tablet form, and (6) coating the tablet with a slow release polymeric coating. The shape of the tablet is not critical.

The granules may be compressed into tablets in a uniform blend of the granules in a single step process of tablet press, or in a layered or segregated manner, in a multi steps tablet press. The layered granules geometry in the tablet press may be horizontal or ex-centuc, wherein a core tablet is made of the minocycline or the antipsychotic drug and the other drug layer or dose encapsulate the inner body.

The granules of minocycline or of the antipsychotic drug may be uniform or not. Non uniform granules of different size or different composition or granules that are further encapsulated enable control on release profile and obtaining continuous or pulsatile release and may be designed for optimal clinical efficacy and patient compliance.

Gastrointestinal disturbances such as nausea and vomiting are sometimes observed in patients treated with tetracyclines, including minocycline, whereas antipsychotics in general have an antiemetic effect. Therefore, in a combined therapy, it would be beneficial for the patient to take advantage of the antiemetic properties of the antipsychotic drug to counter the undesirable gastrointestinal side effects of the tetracycline.

It is now indicated that certain modified release pharmaceutical compositions allow administration of a single daily dose of a typical or an atypical antipsychotic drug, and a tetracycline, to provide an advantageous delivery of drug for the treatment of psychiatric disorders. The combined therapy advantageously provides reduced adverse side effects. Such modified release is therefore considered to be particularly useful for the delivery of antipsychotic drugs in combination with the tetracycline minocycline for use in the treatment of psychiatric disorders, especially schizophrenia. It is expected that reduced side effects and single daily dosage form will improve patient compliance.

Accordingly, a pharmaceutical composition, suitable for the treatment of psychiatric disorders, especially schizophrenia, in a mammal, such as a human, is disclosed, which composition comprises: at least one typical or atypical antipsychotic drug, and a pharmaceutically effective amount of minocycline. The composition can be arranged to provide a modified release of the antipsychotic drug and the tetracycline. The remaining active agent would of course be subject to non-modified release.

According to certain embodiments, the release of both the antipsychotic drug and the tetracycline is suitably modified.

According to other embodiments, it is envisaged that the release of only the antipsychotic drug is modified. It is also envisaged that the release of only the tetracycline is modified.

Suitably, the modified release is delayed, pulsed or sustained release.

In one aspect the modified release is a delayed release.

Delayed release is conveniently obtained by use of a gastric resistant formulation such as an enteric formulation, such as a tablet coated with a gastric resistant polymer, for example Eudragit L100-55. Other gastric resistant polymers include methacrylates, cellulose acetate phthalate, polyvinyl acetate phthalate, hydroxypropyl methylcellulose phthalate, in particular, Aquateric, Sureteric, HPMCP-HP-55S.

The enteric coated tablet may be a single layer tablet, where the active agents are admixed prior to compression into tablet form, or a multi-layer tablet, such as a bi-or tri-layer tablet, wherein each active agent is present in a discrete layer within the compressed tablet form. The discrete table layers can be arranged as required to provide modified or non-modified release of each active agent.

In a further aspect the modified release is a sustained release, for example providing effective release of active agents over a time period of up to 26 hours, typically in the range of 4 to 24 hours.

Sustained release is typically provided by use of a sustained release matrix, usually in tablet form, such as disintegrating, non-disintegrating or eroding matrices.

Sustained release is suitably obtained by use of a non disintegrating matrix tablet formulation, for example by incorporating Eudragit RS into the tablet. Alternative non disintegrating matrix tablet formulations are provided by incorporating methacrylates, cellulose acetates, hydroxypropyl methylcellulose phthalate, in particular Eudragit L and RL, Carbopol 971P, HPMCP-HP-55S into the tablet.

Sustained release is further obtained by use of a disintegrating matrix tablet formulation, for example by incorporating methacrylates, methylcellulose, in particular Eudragit L, Methocel K4M into the tablet.

Sustained release can also be achieved by using a semi-permeable membrane coated tablet for example by applying methacrylates, ethylcellulose, cellulose acetate, in particular Eudragit RS, Surelease to the tablet.

Sustained release can also be achieved by using a multi layer tablet, where each active ingredient is formulated together or as a separate layer, for example as a matrix tablet, with the other layers providing further control for sustained release of either one or both active agents.

Sustained release can also be achieved by using implants.

In yet a further aspect the modified release is a pulsed release, for example providing up to 4, for example 2, pulses of active agent per 24 hours.

One form of pulsed release is a combination of non-modified release of active agent and delayed release.

Suitable modified release includes controlled release. The composition of the invention also envisages a combination of pulsed, delayed and/or sustained release for each of the active agents, thereby enabling for example the release of the reagents at different times. For example, where the composition comprises an antipsychotic drug and a tetracycline, such as minocycline, the composition can be arranged to release the minocycline overnight.

A suitable typical antipsychotic is haloperidol. Other suitable typical antipsychotic drugs are chlorpromazine, chlorprothixene, fluphenazine, loxapine, mesoridazine, perphenazine, pimozide, thioridazine, thiothixene, trifluoperazine, and trifluopromazine.

Suitable atypical antipsychotic drugs include olanzapine, risperidone, clozapine, quetiapine, paliperidone, ziprasidone, and aripiprazole.

Suitable tetracyclines include tetracycline, aureomycine, terramycine, demethylchlortetracycline, rolitetracycline, methacycline, doxycycline, and glycylcyclines. A preferred is tetracycline is minocycline.

It will be understood that the antipsychotic drugs and the tetracyclines are in a pharmaceutically acceptable form, including pharmaceutically acceptable derivatives such as pharmaceutically acceptable salts, esters and solvates thereof, as appropriate to the relevant pharmaceutically active agent chosen. In certain instances herein the names used for the antipsychotic drug or the tetracycline may relate to a particular pharmaceutical form of the relevant active agent: it will be understood that all pharmaceutically acceptable forms of the active agents per se are encompassed by this invention.

Suitable pharmaceutically acceptable forms of the antipsychotic drugs and tetracyclines depend upon the particular agent used but included are known pharmaceutically acceptable forms of the particular agent chosen. Such derivatives are found or are referred to in standard reference texts such as the British and US Pharmacopoeias, Remington's Pharmaceutical Sciences (Mack Publishing Co.), The Extra Pharmacopoeia (London, The Pharmaceutical Press).

The administration of the composition occurs at fixed intervals, usually once to thrice daily. The administration pattern will depend on the compound or compounds administered and the required dosage, and can easily be determined by the skilled therapist. Alternatively, the administration occurs at variable intervals. In these cases, the time between administrations will vary according to the general condition of the patient, and will be decided by the therapist.

The daily dose of minocycline can be administered in a single dose once a day, or in several smaller doses at least twice daily at fixed or variable intervals.

Since some combinations used to treat the psychotic disorders comprise at least two active ingredients, which may be administered separately, also disclosed but not forming part of the present invention is a kit comprising a pharmaceutical composition comprising the tetracycline component of the combination in a first unit dosage form, and a pharmaceutical composition comprising the antipsychotic component in a second unit dosage form. The kit also includes container means for containing the separate compositions such as a divided bottle or divided foil packet. However, the separate compositions may also be contained within a single undivided container. The kit form is particularly advantageous when the separate components are preferably administered through different routes (e.g., orally and parenterally), in different pharmaceutical formulations (e.g., in the form of tablets and syrups), at different dosage, intervals, or when the prescribing physician desires titration of the combination.

The clinical onset of schizophrenia is preceded by subtle signs that a specialist is able to recognize. For example, first-degree relatives of schizophrenic patients are at higher risk to develop schizophrenia than the general population, and might be followed for the appearance of such signs. Minocycline can be administered in dosage amounts of about 10 mg/day to about 500 mg/day, and preferably of about 50 mg/day to about 200 mg/day.

The daily dose of minocycline can be administered in a single dose once a day, or in several smaller doses at least twice daily at fixed or variable intervals.

The present invention presents evidence of the effects of minocycline on cognitive deficits caused by MK108, a compound used in an animal model of schizophrenia. The ability of minocycline to counter the cognitive disruptive effect of MK108, was tested in tasks assessing visual-spatial memory (Morris Water Maze) and sensory-gating performance (the Acoustic Startle Response and Pre-Pulse Inhibition paradigms). The findings indicate that MK801 caused cognitive deficits in visuo-spatial memory abilities, pre-attentional information processing and sensorimotor gating. Minocycline significantly and differentially reversed the cognitive deficits induced by MK801. Moreover, minocycline was more effective than the typical antipsychotic haloperidol in reversing the cognitive deficits. This beneficial effect is especially evident in spatial learning and sensory-gating performance.

Minocycline was also beneficial as an adjuvant drug in antipsychotic therapy in schizophrenic patients Results of an ongoing multi-center, double blind, and comparative prospective fixed dose trial aimed at assessing the effect of minocycline as an adjuvant drug on the progression of the negative signs of schizophrenia are presented. The results show that minocycline improved the negative symptoms and cognitive deficits of schizophrenic human subjects when added as an adjuvant to an antipsychotic drug. A beneficial effect of minocycline was also noted on weight gain during antipsychotic treatment. A significantly lower percentage of patients treated with minocycline gained weight 12 weeks after start of the treatment compared to patients receiving the placebo, while a higher percentage of patients treated with minocycline lost weight compared to patients receiving the placebo.

The present invention will now be illustrated by the following examples which are intended to be construed in a non-limitative fashion.

### EXAMPLES

### Examples 1-4: Effectiveness of minocycline in an animal model of schizophrenia

### Materials and Experimental Apparatus

**Dizocilpine maleate** (MK801): Fresh solutions of dizocilpine maleate (MK801) (Sigma-Aldrich, Israel) were prepared in physiological saline (0.9% NaCl in sterile distilled water) for each batch of rats.

**Minocycline:** Minocycline hydrochloride (Sigma, St. Louis, MO) (35 mg/kg) was freshly dissolved in phosphate-buffered saline (KPBS, pH 7.2, 37 °C).

**Haloperidol:** Haloperidol (0.4 mg/kg; Sigma-Aldrich, Israel) was dissolved with a minimal amount of glacial acetic acid, around 10 µl, and then diluted with lukewarm 5.5% D-glucose, with a final pH around 6.0.

**Saline:** Saline was used for control injections.

**Morris Water Maze (MWM):** The MWM consisted of a water pool (diameter=1.8 m; depth=0.6 m) containing water at 26±1°C. A hidden platform was located 1.5 cm below the water surface. Within the testing room, only distal visuo-spatial cues were available to the rats for location of the submerged platform. A video camera was placed above the center of the pool for tracking the rat, and a video tracking system (Noldus Information Technology b.v., Wageninpen, The Netherlands) with online digital output, directly fed data into a computer and analyzed. Data were analyzed by using an Etho-Vision automated tracking system (Noldus Information Technology).

**Acoustic Startle Response (ASR) and Pre-Pulse Inhibition (PPI):** Both experimental paradigms were performed in a room that was adjacent to the laboratory facilities and the animal stall and that was not used for other experiments. The animals were tested in squad of two with startle chambers counterbalanced across the different experimental groups. Startle response and prepulse inhibition were measured using two ventilated startle chambers (SR-LAB system, San Diego Instruments, San Diego, CA). Each chamber consisted of a Plexiglas cylinder resting on a platform inside a sound-attenuated, ventilated chamber. A high-frequency loudspeaker inside the chamber produced both a continuous broadband background noise of 70 dB and the various acoustic stimuli. Movement inside the tube was detected by a piezoelectric accelerometer below the frame. The amplitude of the whole body startle to an acoustic pulse was defined as the average of 100 one-msec accelerometer readings collected from pulse onset. These signals were then digitized and stored by a computer. Sound levels within each test chamber were measured routinely using a sound level meter (Radio Shack) to ensure consistent presentation. The SR-LAB calibration unit was used routinely to ensure consistent stabilimeter sensitivity between test chambers and over time (Swerdlow and Geyer, 1998, Schizophrenia Bulletin, 24(2), 285-301).

### Experimental Methods and Data Analysis

### Main experimental procedure:

Rats (N=50) were injected with minocycline (35mg/kg) for three consecutive days before the behavioral testing. Thirty minutes before behavioral assessments rats were either injected intraperitoneally (i.p.) with haloperidol (groups; haloperidol, haloperidol+MK801) or saline (groups; minocycline, saline, minocycline+MK801, MK801), a time frame effective in inducing a behavioral effect in rodents (see material section for dose data). Fifteen minutes before the behavioral procedures conducted at the fourth day, the subject was injected i.p. with an 0.1 mg/kg dose of MK801 or saline, for a total volume of 1ml per kg of body weight. Next, the subjects passed the three behavioral procedures, described in the following paragraphs.

### The Morris Water Maze (MWM) paradigm:

The MWM, a hippocampal-dependent visuo-spatial learning task, was used to assess the subjects' spatial learning/memory. The ability of an animal to locate a hidden platform submerged under water by using extra-maze cues from the test environment was examined. The rats were trained in a pool 1.8 m in diameter and 0.6 m high, containing water at 26±1°C. A 10 cm square transparent platform was hidden in a constant position in the pool submerged 1 cm below the water level. Within the testing room only distal visuo-spatial cues were available to the rats for location of the submerged platform.

Rats were given four trials per day, to find the hidden platform (acquisition phase). The escape latency, i.e., the time required by the rat to find and climb onto the platform, was recorded for up to 120 sec. Each rat was allowed to remain on the platform for 30 sec, and was then removed to its home cage. If the rat did not find the platform within 120 sec, it was manually placed on the platform and returned to its home cage after 30 sec.

### Acoustic Startle Response (ASR) and Pre-Pulse Inhibition (PPI) paradigms:

ASR and PPI were measured using two startle chambers, with each chamber consisting of a Plexiglas cylinder resting on a platform inside a sound-attenuated, ventilated chamber (see also materials section). The animals were tested in pairs with startle chambers counterbalanced across the different experimental groups.

The ASR test measured the ability of the animal to habituate to repetitive loud pulses of noise. At the beginning of the experiment, the animals were placed inside the tube and the startle session started with a 5-min acclimatization period (during which a high-frequency loudspeaker inside the chamber produced a continuous broadband background noise of 68 dB). Following the acclimatization period, four startle pulses (30 ms, 120 dB) were presented. Movement inside the tube was detected by a piezoelectric accelerometer below the frame. The amplitude of the whole body startle to an acoustic pulse was defined as the average of 100 one-msec accelerometer readings collected from pulse onset. These signals were then digitized and stored by a computer. The SR-LAB calibration unit was used routinely to ensure consistent stabilimeter sensitivity between test chambers and over time.

PPI of the startle response was used as a measure of sensoumotor gating, with the startle chambers used to assess the ability of prepulse stimuli to block the startle response. The prepulses were noise bursts of either 72, 76, 80, or 84 dB and were 20 msec in duration. The interval between the prepulse and the startle pulse was 80 msec. Each session consisted of six blocks of 10 trials. Each block included four different trial types: two startle pulse alone trials, four pre-pulses at different intensities, followed by startle pulse, four prepulses alone at four intensities, and one no-stimulus trial. The different trial types were presented pseudo-randomly with a variable inter-trial interval of 10-20 sec.

### Data Analysis

**Morris Water Maze (MWM):** Escape latency, the time between placing the rat in the pool until it located the platform, was measured during behavioral testing. The escape latency was analyzed by means of three-way repeated measures analysis of variance (ANOVA) with a between-subjects measure of 'group' (treatment group) and a within-subjects measure of 'trail' (effects amongst groups in the four different trials). Two separate analyses were conducted for the minocycline dose determination [comparing; MK801, saline, and four minocycline receiving groups (20, 25, 30, 35 mg/kg)] and the main experimental procedure [comparing; MK801, saline, minocycline (35mg/kg), minocycline+MK801, haloperidol and haloperidol+MK801].

**Acoustic Startle Response (ASR):** The decrease in ASR in the groups was calculated as the percent of amplitude decrease between the mean of the first five amplitude values and the mean of the last 25 amplitude values.

Percent habituation= 100x[(average startle amplitude in Block 1)-(average startle amplitude in Block 5) / (average startle amplitude in Block 1)]. Results were analyzed by two-way ANOVA a between-subject measure of 'group' (treatment group).

**Pre-Pulse Inhibition (PPI):** Startle magnitude is expressed in all the procedures as arbitrary units. Percent PPI (% PPI) was calculated as the mean startle magnitude to stimulus-alone minus the mean startle magnitude to prepulse-stimulus trials, all divided by the mean stimulus-alone trials, and multiplied by 100. The overall mean % PPI was calculated for the four prepulse intensities (72, 74, 80, 84 dB). Results were analyzed by two-way ANOVA with a between-subject measure of 'group' (difference the treatment groups) and 'prepulse intensity' (72, 76, 80, 84 dB).

### Example 1

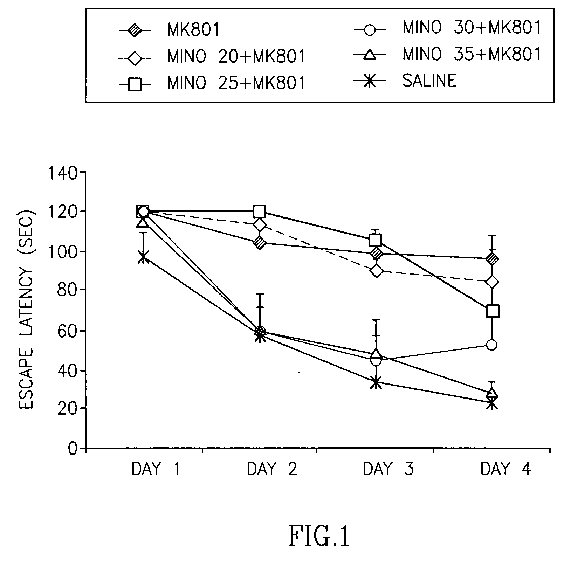

**Determination of minocycline dose effective in reversing the MK801 effects.** Rats were treated for three consecutive days with one of four doses of minocycline (20, 25, 30, and 35 mg/kg) or saline before being injected with MK801 or saline on the fourth day. The MWM test was then performed (see Fig. 1 for results). A significant main effect of 'group', 'trail' and a significant 'group' x 'trails' interaction was found in the repeated measured ANOVA for the MWM task [F(5,35)=9.699, p<0.001; F(3,33)=31.551, p<0.001; F(15,91)=1.824, p<0.05; respectively]. The MK801 treated rats showed longer escape latencies (indicating worse performance) compared to the saline group in a post-hoc Scheffe test (p<0.001). The lower doses of minocycline (20 and 25 mg/kg) had a limited effect of significantly longer escape latencies compared to the saline groups, with no significant differences with the MK801 treated rats. In contrast, the 30 and 35 mg/kg minocycline treated rats did not differ from the saline treated group.

In order to choose between the two highest minocycline doses (30 or 35mg/kg) we performed a follow-up one way ANOVA analyses focusing on each MWM trail. While no significant differences were found for *trail 1* [F(5,35)=1.955, n.s.], a significant 'group' main-effect was found in trails 2-4 [F(5,35)=5.176, p<0.001; F(5,35)=6.751, p<0.001; F(5,35)=7.330, p<0.001; respectively]. Post-hoc Scheffe analyses pointed towards an increasing effect of the pharmacological manipulations; trail 2 indicated no significant differences between treatment groups. In trail 3 only the saline treated rats showed significantly better performance versus the MK801 treatment group (p<0.01). Finally, in trail 4 only the minocycline 35mg/kg treated rats showed a significant difference when compared to the MK801 group (p<0.05) (besides the saline groups that showed a significant difference from the MK801 group; p<0.001). Overall, minocycline showed an increasing effect in the MWM task with each trail accompanied by a good dose dependent effect. The findings suggest that the 35mg/kg dose may be the most suitable to test the ability of minocycline to counter the effects of MK801, without the risk of side-effects possibly induced by higher doses.

### Example 2

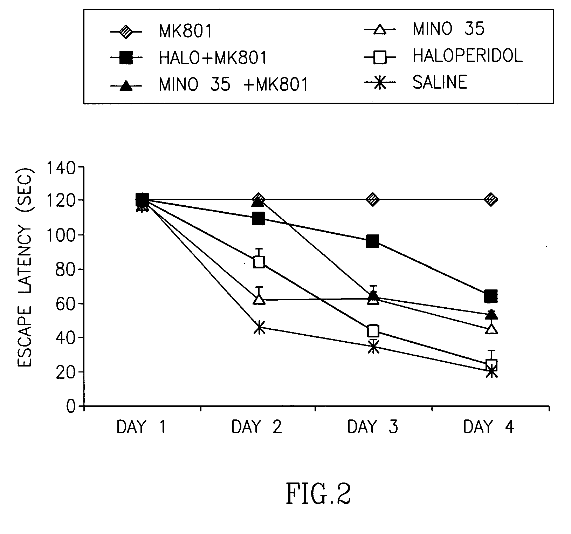

**Comparison of minocycline and haloperidol treatment in the Morris Water Maze (MWM) task.** The main experimental procedure was performed before the MWM test (see Fig. 2 for results). The escape latency (mean ± SEM) of the six treatment groups in the 4 MWM trails was measured and averaged over the experiment days. No differences were found in the first trail of the MWM, trails 2-4 (corresponding to day 2-4) pointed toward longer escape latencies and worse performance of the MK801 treated rats, when compared to most other groups. Minocycline was shown to reduce the impairments induced by MK801, and the improvement somewhat more significant than that caused by haloperidol.

### Example 3

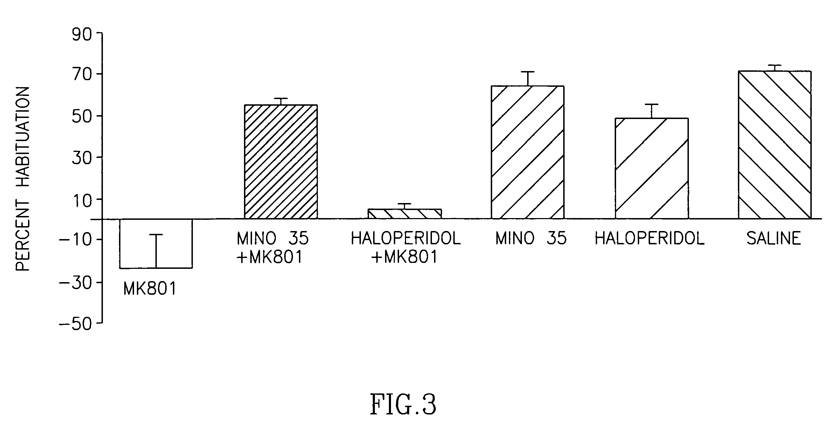

**Effects of minocycline on MK801 induced habituation of the Acoustic Startle Response (ASR)**. The main experimental procedure was performed before the ASR test (see Fig. 3 for results). Habituation was defined as the decrement in startle response to an initially novel stimulus when presented repeatedly (at slow rates in order to avoid sensory adaptation or effector fatigue). A one-way ANOVA revealed a significant 'group' main-effect on the percent habituation of the startle response (% habituation) [F(5,24)=22.4, p<0.001]; post-hoc Scheffe analysis indicated that treatment groups ASR results can be divided into two clusters; (1) MK801 and haloperidol+MK801 groups. (2) Saline, minocycline, haloperidol and minocycline+MK801 treatment groups. The first cluster groups showed over all lower % habituation (lower performance) in the ASR task compared to groups from the second cluster. The MK801 treated rats showed lower % habituation compared to the saline, minocycline, haloperidol and minocycline+MK801 groups (p<0.001). The haloperidol+MK801 group showed lower % habituation (lower performance) when compared to saline (p<0.001), minocycline (p<0.01), minocycline+MK801 (p<0.01) and haloperidol alone (p<0.05) group.

In summary, the findings indicate that MK801 induced a significant deficit in the habituation of ASR in the rats, compared with saline treated rats. Minocycline pretreatment was able to prevent the deficits induced by MK801 and had no effect on habituation of ASR, when compared to the saline treated animals. In contrast, administration of haloperidol was not able to prevent the deficits induced by MK801.

### Example 4

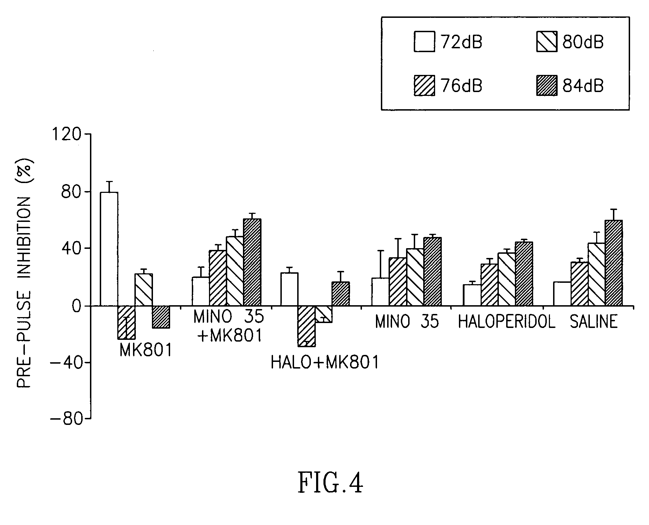

**Sensorimotor gating function evaluated by the Pre-Pulse Inhibition (PPI) paradigm.** The main experimental procedure was performed before the PPI test (see Fig. 4 for results). There were significant 'group' main-effect on prepulse inhibition in the repeated-measures ANOVA for percent of PPI (%PPI) [F(5,24)=15.91, p<0.001]; Scheffe post-hoc tests indicated that groups formed two clusters, similarly to the ASR test (see Example 3): (1) MK801 and haloperidol+MK801 groups (p<0.01 and p<0.001; respectively). (2) saline, minocycline, haloperidol and minocycline+MK801 treatment groups; the latter groups showing higher %PPI and better performance. The ANOVA also showed a significant 'pre-pulse intensity' (72, 276, 80 and 84 dB) main-effect [F (3,72)=8.59, p<0.001]; %PPI was significantly shortened with each trail, showing the expected pre-pulse inhibition of ASR. Finally, there was a significant 'group' x 'prepulse intensity' interaction [F(15,72)=8.50, p<0.001].

There were no statistically significant differences between the saline, minocycline and minocycline+MK801 treated rats, with all groups showing a similar monotonic increase in %PPI in increase in stimulus intensity (significant main effect of 'prepulse intensity' on %PPI for each group; saline- p<0.01; minocycline- p<0.05; minocycline+MK801- p<0.01). In contrast, the MK801 and haloperidol+MK801 did not show a monotonic increase in %PPI, indicating a disruption of PPI.

In summary, MK801 induced a significant deficit in the PPI when compared to the saline treatment group. Minocycline was able to reverse the deficits caused by the MK801 treatment in contrast to the lack of significant effects by the haloperidol (haloperidol+MK801 group).

### Examples 5-12: Effectiveness of minocycline as an adjuvant drug in a study of patients experiencing a first psychotic episode

### Study design:

The study is a multi-center, double blind, and comparative prospective fixed dose trial aimed at assessing the effect of minocycline as an adjuvant drug on the progression of the negative signs of schizophrenia. The study was carried out in patients who experience first psychotic episode, and has followed the patient for a total duration of 6 months. As the study examines Minocycline as an adjuvant drug, the sample includes patients receiving one of the following atypical anti-psychotic drugs: Risperdal, Zyprexa, Geodon, Seroquel, and Leponex. Minocycline's possible effect on various clinical (CGI, PANSS etc.) and cognitive parameters (which may serve as predictors to cognitive deterioration of schizophrenia) was periodically assessed.

In order to differentiate patients with schizophrenia from patients with other disorders (e.g.- brief psychotic episode, schizophreniform disorder, bipolar disease), a repeated Structured Clinical Interview for DSM-IV (SCID) needs to be carried out at the end of the study period in each of the patients.

### Inclusion criteria include:

1. Males or females suffering from a first episode of non-organic acute psychosis;
2. Subject was screened not more than 3 years prior to development of acute psychosis and found to have no sign of major psychopathology;
3. Baseline positive and negative symptom scale (PANSS)>60;
4. Has given verbal assent and has signed the informed consent form.

### Exclusion criteria include:

1. Psychotic episode with predominating affective symptoms;
2. Mental retardation;
3. Acute, unstable, significant or untreated medical illness;
4. Pregnant or breast-feeding females;
5. Drug or alcohol abuse;
6. Any known contraindication to the given drugs.

### Drop-out during study:

Patients are excluded from the study during the study period in case of:
1. Any serious adverse reaction (to either minocycline or the atypical antipsychotic);
2. Clinical deterioration (as measured by increase in 20% in the BPRS for two weeks);
3. Any addition of an anti-psychotic medication other than the drugs mentioned above;
4. Ness increase in the dosage of antipsychotic drugs in the last month.

### Initial and periodical evaluation

Initial evaluation included the following clinical measures:
- SCID (for reliability of diagnosis)
- CGI (Clinical Global Impression)
- PANSS (Positive and Negative Syndrome Scale for Schizophrenia)
- SANS (Scale for the Assessment of Negative Symptoms)
- Calgary Depression Scale for Schizophrenia
- GAF
- GRAF
- Insight and Treatment Attitudes Questionnaire
- QLS (Quality of Life Scale)
- Multinomah Community Ability Scale
- Extrapyramidal Side Effects Scale.

In addition, the subjects were assessed by a battery of computerized neuropsychological tests (CANTAB - Cambridge Neuropsychological Test Automated Battery), measuring attention, memory, and planning.

**Memory** was tested using spatial and pattern recognition and spatial span tasks of the CANTAB.

**Executive function** was tested by two tasks of the CANTAB: planning was tested by a computerized modification of the Tower of London, and working memory by a spatial working memory task.

### Treatment

Minocycline was given as an add-on therapy. Prior to randomization into minocycline/placebo adjuvant treatment, a preliminary (lead-in phase) screening phase of placebo treatment was undertaken: Placebo was given as an adjuvant to all patients for two weeks. Subjects who showed substantial reaction to the placebo treatment (a decrease of >20% in PANSS) were excluded from the study. Randomization was then carried out by the principal investigator. The raters and evaluators of the subjects participating in the study were blind to the randomization. Randomization was to one of two treatment groups: atypical antipsychotic drug + minocycline (200 mg/day) or atypical antipsychotic drug + placebo.

Clinical status was evaluated by the aforementioned scales at the onset of the study, every week for the first 6 weeks, and then once a month for the rest of the study period. Cognitive capabilities were assessed by the neuropsychological battery of tests according to the following schedule: upon initiation of the study (referred to as visit 1 in figures 5-8), 3 months (referred to as visit 2 in figures 5-8), and 6 months (referred to as visit 3 in figures 5-8) after the start of the study. The trial lasted for 28 weeks. Changes in the dosage of the atypical antipsychotic drug, as long as they were in the range, did not exclude the subject. At the end of the study period, all subjects were re-diagnosed by a repeated SCID.

### Data Analysis

Out of 70 subjects recruited, a total of 19 subjects completed the full 28-weeks protocol so far. The ANOVAs for age, age at first hospitalization, number of hospitalizations, and number of education years revealed no significant differences between the minocycline and placebo groups.

The following results are based on analysis of the data of 55 subjects (35 received minocycline, 20 received placebo) who performed at least one computerized cognitive assessment. We used the EM statistical method to complete empty cells of drop-out subjects.

### Examples 5-8: Executive Functions, Working Memory and Planning Tests (the CANTAB test battery)

### Example 5

**Intra-extra dimensional set-shifts (IED).** This task assesses rule acquisition and attentional set shifting abilities. IED is sensitive to cognitive changes associated with schizophrenia. The MANOVA for IED measures (*number of stages completed, total errors adjusted, Pre-ED errors, EDS errors)* showed a significant *time x group* interaction (p<0.0001) (Fig. 5). Follow up ANOVAs indicated that the minocycline group had improved significantly more than the placebo group on all the IED measures except for the Pre-ED errors measure. This indicates that the minocycline group showed a specific improvement in attentional set shifting abilities, rather than in rule acquisition.

### Example 6

**Stockings of Cambridge (SOC) -** This is a spatial planning test based upon the 'Tower of London' test. This test gives a measure of frontal lobe function. The MANOVA for SOC measures (*mean initial thinking time for 5 moves stages, mean subsequent thinking time for 5 moves stages, Number of problems solved in minimum moves)* showed a significant *time x group* interaction (p<0.01) (Fig 6). Follow up ANOVAs indicated that the minocycline group had improved significantly more than the placebo group in the *number of problems solved in minimum moves* measure (p=0.001), i.e. the minocycline group had a greater improvement then the placebo in planning abilities.

### Example 7

**Spatial Working Memory (SWM) -** The task has been linked to the frontal lobe and its sub-divisions (such as the dorsolateral and ventrolateral frontal), and may be considered an additional test of frontal activity. The MANOVA for SWM measures (*between errors, strategy*) showed a significant *time x group* interaction (p<0.0001) (Fig. 7). Follow up ANOVAs revealed that the minocycline group had improved significantly more than the placebo group in the *between errors* measure (p<0.0001) and in the *strategy* score (p<0.05). These results indicate that the minocycline group, compared with the placebo group, showed a greater improvement in the task in terms of efficiency and systematic performance.

### Example 8

**Spatial Recognition Memory (SRM) -** this task is a test of spatial recognition memory, with performance on this task correlated with medial temporal lobe and parietal lobe functions (Milner et al., Philos Trans R Soc Lond B Biol Sci. 1997 ;352:1469-74.; ). The ANOVA for *percent of correct responses* showed a significant *time x group* interaction (p<0.01) (Fig. 8). The minocycline group kept relatively steady scores on this measure, while the placebo group's performance deteriorated.

### Example 9

**SANS (Scale for the Assessment of Negative Symptoms):** ANOVA for *total SANS score* revealed a significant *group x time* interaction (p<0.01) (Fig. 9). The Minocycline group showed greater improvement than the placebo group in the negative symptoms of schizophrenia.

### Example 10

**Calgary Depression Scale for Schizophrenia:** ANOVA for *Total Calgary Score* approached a significant interaction of *group x time* (p=0.099, n.s.) (Fig 10). The minocycline group tended to reach greater improvement in this measure than the placebo group.

### Example 11

**GRAF:** ANOVA for *GRAF score* revealed a significant *group x time* interaction (p<0.05) (Fig. 11). The minocycline group showed greater improvement of the GRAF score compared to the placebo group.

### Example 12

The body weight of the patients was measured at the start of the study and then 12 weeks later (Fig. 12). A higher proportion of patients lost weight in the minocycline group (46%) compared to the placebo group (33%), while a lower proportion of patients gained weight in the minocycline group (31%) compared to the placebo group (67%). In summary, minocycline treatment had a beneficial effect on weight gain.

### Example 13

An experimental study using the MK801 animal model of schizophrenia has been designed to test the in vivo efficiency of the combination between antipsychotic and minocycline. The experimental results will be used to determine the best regimen in terms of treatment timing. The experimental protocol is presented hereinbelow.
Animals: adult male Sprague-Dawley rats
Number of animals: 6-8 rats per group
Materials: dizocilpine maleate MK801 (Sigma-Aldrich, Israel); Minocycline hydrochloride (Sigma, St. Louis, MO); Halopeudol (Sigma-Aldrich, Israel)
Experimental Behavioral Tests: Morris Water Maze (MWM); Acoustic Startle Response (ASR) and Pre-Pulse Inhibition (PPI)

### Protocol:

### 1. Determine synergy of antipsychotic and minocycline by testing various dosage regimens.

### Test groups:

1. MK801 alone
2. MK801 + haloperidol (0.30 mg/kg) + minocycline (30 mg/kg)
3. MK801 + haloperidol (0.25 mg/kg) + minocycline (25 mg/kg)
4. MK801 + haloperidol (0.20 mg/kg) + minocycline (20 mg/kg)

### 2. Based on above dosage, determine the best regime in terms of treatment timing.

### Test groups:

1. MK801 alone
2. MK801 + haloperidol (time 0) + minocycline (time -6h)
3. MK801 + haloperidol (time 0) + minocycline (time -3h)
4. MK801 + haloperidol (time 0) + minocycline (time 0h)
5. MK801 + haloperidol (time 0) + minocycline (time +3h)
6. MK801 + haloperidol (time 0) + minocycline (time +6h)

## Claims

1. A pharmaceutical composition comprising as active ingredients a pharmaceutically effective amount of minocycline and a pharmaceutically effective amount of at least one antipsychotic drug for use in the treatment of a first psychotic episode of a psychotic disorder.

2. The pharmaceutical composition for use of claim 1, wherein the psychotic disorder is selected from the group consisting of: schizophrenia, bipolar disorder, mood disorders, post-traumatic disorder, eating disorders, obsessive-compulsive disorder, substance-related disorders, somatoform disorders, and micro psychotic episodes in personality disorders, preferably wherein the disorder is schizophrenia

3. The pharmaceutical composition for use according to claim 1 and 2, wherein the antipsychotic drug is a typical antipsychotic.

4. The pharmaceutical composition for use according to claim 3, wherein the typical antipsychotic drug is selected from the group consisting of: haloperidol, chlorpromazine, chlorprothixene, fluphenazine, loxapine, mesoridazine, perphenazine, pimozide, thioridazine, thiothixene, trifluoperazine, and trifluopromazine.

5. The pharmaceutical composition for use according to claim 1 and 2, wherein the antipsychotic is an atypical antipsychotic.

6. The pharmaceutical composition for use according to claim 5, wherein the atypical antipsychotic is selected from the group consisting of: olanzapine, risperidone, clozapine, quetiapine, paliperidone, ziprasidone, and aripiprazole.

7. The pharmaceutical composition for use according to claim 1 and 2, wherein the formulation is a modified release formulation.

8. The pharmaceutical composition for use according to claim 7, wherein the modified release formulation is selected from the group consisting of: a delayed release formulation, a sustained release formulation, a controlled release formulation, a pulsed release formulation, and any combination thereof.

9. The pharmaceutical composition for use according to claim 7, wherein the release of both the minocycline and the antipsychotic drug is a modified release.

10. The pharmaceutical composition for use according to claim 7, wherein only the release of the minocycline is modified.

11. The pharmaceutical composition for use according to claim 7, wherein only the release of the antipsychotic drug is modified.

12. The pharmaceutical composition for use according to claim 7, wherein the antipsychotic drug is released substantially before the tetracycline.

## Patentansprüche

1. Pharmazeutische Zusammensetzung, welche als aktiven Inhaltsstoff eine pharmazeutisch wirksame Menge of Minozyklin und eine pharmazeutisch wirksame Menge mindestens eines anti-psychotischen Arzneimittels zur Verwendung bei der Behandlung einer ersten psychotischen Episode einer psychotischen Störung.

2. Pharmazeutische Zusammensetzung zur Verwendung nach Anspruch 1, worin die psychotisch Störung ausgewählt ist aus der Gruppe bestehend aus: Schizophrenie, bipolarer Störung, Gemüts-Störungen, post-traumatischen Störung, Ess-Störungen, obsessivkompulsiver Störung, Substanz-assoziierter Störungen, somatoform Störungen, und mikropsychotischen Episoden in Persönlichkeitsstörungen, vorzugsweise worin die Störung Schizophrenie ist.

3. Pharmazeutische Zusammensetzung zur Verwendung nach Anspruch 1 und 2, worin das anti-psychotische Arzneimittel ein typisch antipsychotisches Mittel ist.

4. Pharmazeutische Zusammensetzung zur Verwendung nach Anspruch 3, worin das typisch anti-psychotische Arzneimittel ausgewählt ist aus der Gruppe bestehend aus: Haloperidol, Chlorpromazin, Chlorprothixen, Fluphenazin, Loxapin, Mesoridazin, Perphenazin, Pimozid, Thioridazin, Thiothixen, Trifluoperazin und Trifluopromazin.

5. Pharmazeutische Zusammensetzung zur Verwendung nach Anspruch 1 und 2, worin das antipsychotisch Mittel ein atypisches anti-psychotisches Mittel ist.

6. Pharmazeutische Zusammensetzung zur Verwendung nach Anspruch 5, worin das atypische antipsychotische Mittel ausgewählt ist aus der Gruppe bestehend aus : Olanzapin, Risperidon, Clozapin, Quetiapin, Paliperidon, Ziprasidon und Aripiprazol.

7. Pharmazeutische Zusammensetzung zur Verwendung nach Anspruch 1 und 2, worin die Formulierung eine Formulierung mit modifizierter Freisetzung ist.

8. Pharmazeutische Zusammensetzung zur Verwendung nach Anspruch 7, worin die Formulierung mit modifizierter Freisetzung ausgewählt ist aus der Gruppe bestehend aus: einer Formulierung mit verzögerter Freisetzung, einer Formulierung mit verlängerter Freisetzung, einer Formulierung mit gesteuerter Freisetzung, einer Formulierung mit pulsierender Freisetzung und jeder Kombination davon.

9. Pharmazeutische Zusammensetzung zur Verwendung nach Anspruch 7, worin die Freisetzung von Minozyklin und dem anti-psychotischen Arzneimittel eine modifizierte Freisetzung ist.

10. Pharmazeutische Zusammensetzung zur Verwendung nach Anspruch 7, worin nur die Freisetzung des Minozyklin modifiziert ist.

11. Pharmazeutische Zusammensetzung zur Verwendung nach Anspruch 7, worin nur die Freisetzung des anti-psychotischen Arzneimittels modifiziert ist.

12. Pharmazeutische Zusammensetzung zur Verwendung nach Anspruch 7, worin das anti-psychotische Arzneimittel im Wesentlichen vor dem Tetrazyklin freigesetzt wird.

## Revendications

1. Composition pharmaceutique comprenant, en tant que principes actifs, une quantité pharmaceutiquement efficace de minocycline et une quantité pharmaceutiquement efficace d'au moins un médicament antipsychotique pour une utilisation dans le traitement d'un premier épisode psychotique d'un trouble psychotique.

2. Composition pharmaceutique pour une utilisation selon la revendication 1, dans laquelle le trouble psychotique est sélectionné dans le groupe constitué par la schizophrénie, un trouble bipolaire, des troubles de l'humeur, un trouble post-traumatique, des troubles de l'alimentation, un trouble obsessionnel compulsif, des troubles liés à des substances, des troubles somatoformes et des épisodes micropsychotiques dans des troubles de la personnalité, de préférence dans laquelle le trouble est la schizophrénie.

3. Composition pharmaceutique pour une utilisation selon la revendication 1 ou 2, dans laquelle le médicament antipsychotique est un antipsychotique typique.

4. Composition pharmaceutique pour une utilisation selon la revendication 3, dans laquelle le médicament antipsychotique typique est sélectionné dans le groupe constitué par l'halopéridol, la chlorpromazine, le chlorprothixène, la fluphénazine, la loxapine, la mésoridazine, la perphénazine, le pimozide, la thioridazine, le thiothixène, la trifluopérazine et le trifluopromazine.

5. Composition pharmaceutique pour une utilisation selon la revendication 1 ou 2, dans laquelle le médicament antipsychotique est un antipsychotique atypique.

6. Composition pharmaceutique pour une utilisation selon la revendication 5, dans laquelle l'antipsychotique atypique est sélectionné dans le groupe constitué par l'olanzapine, la rispéridone, la clozapine, la quétiapine, la palipéridone, la ziprasidone et l'aripiprazole.

7. Composition pharmaceutique pour une utilisation selon les revendications 1 et 2, dans laquelle la formulation est une formulation à libération modifiée.

8. Composition pharmaceutique pour une utilisation selon la revendication 7, dans laquelle la formulation à libération modifiée est sélectionnée dans le groupe constitué par une formulation à libération retardée, une formulation à libération prolongée, une formulation à libération contrôlée, une formulation à libération pulsée et n'importe quelle combinaison de celles-ci.

9. Composition pharmaceutique pour une utilisation selon la revendication 7, dans laquelle la libération de la minocycline ainsi que du médicament antipsychotique est une libération modifiée.

10. Composition pharmaceutique pour une utilisation selon la revendication 7, dans laquelle seule la libération de la minocycline est modifiée.

11. Composition pharmaceutique pour une utilisation selon la revendication 7, dans laquelle seule la libération du médicament antipsychotique est modifiée.

12. Composition pharmaceutique pour une utilisation selon la revendication 7, dans laquelle le médicament antipsychotique est libéré sensiblement avant la tétracycline.
